# EUROPEAN PATENT SPECIFICATION

(11) **EP 3 554 597 B1**
(45) Date of publication and mention of the grant of the patent: **21.10.2020**
(21) Application number: 17826060.0
(22) Date of filing: 14.12.2017
(51) Int. Cl.: A61M 11/04, A24F 40/00, A24F 40/48, A61M 15/00, A61M 16/00

(54) **VAPOR PRODUCTION DEVICE, SYSTEM, METHOD FOR PRODUCING INHALABLE VAPOR, AND KIT**
DAMPFERZEUGUNGSVORRICHTUNG, SYSTEM, VERFAHREN ZUR HERSTELLUNG VON INHALIERBAREM DAMPF, UND KIT
DISPOSITIF DE PRODUCTION DE VAPEUR, SYSTÈME, PROCÉDÉ DESTINÉ À PRODUIRE DE LA VAPEUR INHALABLE, ET KIT

(30) Priority: 14.12.2016 US 201662433995 P; 13.04.2017 US 201762485216 P
(43) Date of publication of application: 23.10.2019
(73) Proprietor: VMR Products LLC, San Francisco, CA 94107 (US)
(72) Inventor: VERLEUR, Jan, Andries, San Francisco, CA 94107 (US); RECIO, Dan, San Francisco, CA 94107 (US); LIU, Zhiyuan, San Francisco, CA 94107 (US); VERLEUR, Hans, San Francisco, CA 94107 (US)
(74) Representative: Thum, Bernhard
(86) International application number: PCT/US2017/066367
(87) International publication number: WO 2018/112178

(56) References cited:
- EP-A2- 0 877 196
- WO-A1-99/11311
- US-A- 3 211 191
- US-A- 3 266 674
- US-A- 4 619 297
- US-A1- 2008 216 825
- US-A1- 2014 212 517
- US-A1- 2015 305 409
- US-A1- 2016 331 034
- US-B1- 6 314 957
- US-B1- 6 513 524

## Description

### BACKGROUND

### 1. Technical Field text

This disclosure relates generally to vaporizers, and more particularly to a vapor production system capable of storing vapor.

### 2. Background Information

Vaporizers have recently emerged as a new product for providing nicotine and other products through a smokeless inhalation process. There are many embodiments of vaporizers including the electronic cigarette. Most implementations consist of a power supply (typically a battery) and an atomizing device. In reusable electronic cigarettes the two items are separated into a battery and a cartomizer, to allow the disposal and replacement of the nicotine containing fluid cartomizer while preserving the more costly battery and associated circuitry (microcontroller, switch, indicating LED, etc.) In disposable electronic cigarettes the two items are combined to integrate the functions into one unit that is disposed of after either the battery energy or the nicotine containing E-liquid is exhausted. Vaporizers are generally designed to provide a supply of vapor on demand to a single user. In instances where multiple users are present, each user typically has their own vaporizer.

The E-liquid that is used to produce vapor in electronic cigarettes is generally a solution of one or more of propylene glycol (PG) and/or vegetable glycerin (VG) and/or polyethylene glycol 400 (PEG400) mixed with concentrated flavors, and optionally, a variable percentage of a liquid nicotine concentrate. This liquid may be termed an "E-liquid" and is often sold in a bottle or in disposable cartridges or cartomizers. Many different flavors of such E-liquids are sold, including flavors that resemble the taste of regular tobacco, menthol, vanilla, coffee, cola and various fruits. Various nicotine concentrations are also available, and nicotine-free E-Liquids are also common.

US 2016331034 A1 describes an electronic vapor device comprising: a vapor outlet; a first container for storing a first vaporizable material, wherein the first container is permanently integrated into the electronic vapor device; a second container for storing a second vaporizable material, wherein the second container is removable from the electronic vapor device; a docking bay configured to receive the second container, wherein the second container is removed from or inserted into the docking bay through a door; and a vaporizer component configured for vaporizing the first vaporizable material or the second vaporizable material to generate a vapor and for providing the vapor to the vapor outlet. US 2016331034 discloses, in a separate embodiment: a vaporizer comprising a depressurization chamber, a vapor chamber, a first vapor conduit, a second vapor conduit, and a processor; and a container configured to hold a pre-vaporized liquid, under pressure, wherein the pre-vaporized liquid was generated, pressurized, and stored for later use; for example, a vaporizable element may be vaporized externally to the electronic vapor device; the vapor generated can be captured, compressed, and stored in the container; or, during the manufacture of the vaporizer, the vaporizable element may be created in the vaporizer device itself and compressed, and stored in the container; for example, an aerosol may be formed in carbon dioxide, compressed using equipment for compressing carbon dioxide, and stored in a cartridge suitable for carbon dioxide; in an alternative, pure carbon dioxide or other carrier gas may be pressurized and introduced into a cartridge that already contains a quantity of the material to be aerosolized, for example a powdered or liquid material.

US 2014212517 discloses a device for capturing a component of a vapor, comprising a vaporizer and a vapor trap, the vapor trap in communication with a vaporizer outlet. The vaporizer has an air pump, an air inlet upstream of the air pump, an air heater in communication with the air pump, a vaporization chamber in communication with the air pump and downstream of the air heater, and a vaporizer outlet downstream of the vaporization chamber. In operation, a quantity of a vaporizing material is positioned within the vaporization chamber. The vapor trap forms an interior reservoir and has a vapor inlet and a vapor outlet positioned on opposite sides. A first container is positioned just downstream of the vapor inlet, which is configured to allow a vapor inlet flow and simultaneously prevent escape of the vapor trapping media through the vapor inlet. A second container is positioned just upstream of the vapor outlet, configured to allow a vapor outlet flow and simultaneously to prevent escape of the vapor trapping media through the vapor outlet.

US 6513524 discloses a table-top apparatus with a seated receptacle and a valve as well as a vapor balloon. In the inside of a housing of the apparatus there is a hot air generator comprised of a motor, propeller, heat chamber and air flow tube which draws in the air from below through an air inlet provided in the base, heats and, following the laws of physics, in accordance with which heated air climbs upwards, conveys this upwards ("volcano"-smoke stack). Further there is situated on the outside of the housing a temperature regulator as well as an on/off switch. The receptacle includes a receptacle chamber in which either a plant material to be vaporized is loosely filled in or a crucible with a fluid to be vaporized is introduced. The receptacle further comprises a smoke detector to monitor the vapor upon the development of undesired smoke. The smoke detector provides a signal to a controller to regulate the temperature of the hot air source. The valve is comprised of a light metal block in which a valve cylinder and a valve spring wound on the outside of the valve cylinder are provided. The valve cylinder is rigidly connected with the valve cover via spacers. The openings between the spacers make it possible for the vapor to can travel into and out of the balloon while the valve is opened. Besides this the funnel shape itself insures for a perfect sealing when the valve is closed, and also holds the balloon securely in its place. The vapor balloon is comprised of a balloon jacket and a balloon retainer. Hot air is blown through the plant material and thereby the aroma- and active substances are transitioned into vapor, which is directed into the balloon via the valve and is collected there. The vapor can then be inhaled out of this balloon by means of a mouthpiece connected to the valve.

It is possible to draw the produced vapor/air mixture out of the balloon by means of a compressor for compressing and filling into a pressure container, or to directly fill the produced vapors via a compressor into a pressure container.

### BRIEF SUMMARY

According to the present invention, there is provided a vapor production device according to claim 1, a system according to claim 5, a method according to claim 8 and a kit according to claim 10. Preferred embodiments of the invention are defined in the dependent claims 2-4, 6, 7, 9 and 11-13.

### BRIEF DESCRIPTION OF THE DRAWINGS

FIG. 1 illustrates a schematic view of a vapor compression system according to an embodiment of the invention.
FIG. 2 illustrates a flowchart of a method of producing inhalable vapor, according to an embodiment of the invention.
FIG. 3 illustrates a storage container not being according to an embodiment of the invention.

### DETAILED DESCRIPTION

The following detailed description and the appended drawings describe and illustrate some embodiments of the disclosure for the purpose of enabling one of ordinary skill in the relevant art to make and use these embodiments. As such, the detailed description and illustration of these embodiments are purely illustrative in nature and are in no way intended to limit the scope of the invention, which is defined by the claims. It should also be understood that the drawings are not necessarily to scale and in certain instances details may have been omitted, which are not necessary for an understanding of the embodiments, such as details of fabrication and assembly. In the accompanying drawings, like numerals represent like components.

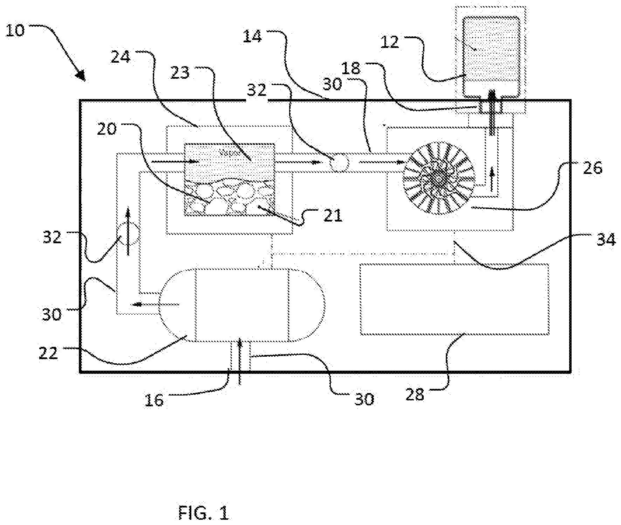

FIG. 1 illustrates a schematic view of a vapor production system 10. Vapor production system 10 is generally a system for producing compressed vapor suitable for filling a container 12 for later consumption by an end user. Once filled by vapor production system 10, container 12 may be used to consume vapor absent the vapor production system 10. Thus, vapor may be available in a setting that may not allow a traditional vaporizer, or in situations in which a high vapor production rate is required.

Vapor production system 10 is contained within a portable case 14. Portable case 14 is powered by way of batteries, an external power source, or a combination of batteries and an external power system. Portable case 14 has an outer surface with an inlet 16 for receiving air, an outlet 18 for produced vapor, and vaporizable material chamber 20 for receiving vaporizable material to be vaporized. The vaporizable material chamber 20 may receive cartridges containing particular types of vaporizable materials, or it may receive the raw vaporizable materials to be vaporized.

Contained within portable case 14 are components for producing vapor. Also contained within some embodiments of the vapor production system or its portable case are components for compressing vapor. In yet other embodiments, components for both producing vapor and for its compression are contained within the vapor production system or its portable case. As shown in FIG. 1, the components includes an air pump 22, a heating center 24, a vapor compressor 26, and a control panel 28. Additionally, each component is in fluid communication with adjacent components though air channels 30 with fluid flow between components controlled by valves 32. Control panel 28 is wired to each component and one or more valves 32 through common techniques as known by one of ordinary skill in the art. Dashed line 34 indicates the connection between control panel 28 and the components.

Air pump 22 receives ambient air through inlet 16. Inlet 16 may be connected directly to air pump 22, or as shown in the embodiment of FIG. 1, inlet 16 may connect to air pump 22 by way of air passage 32. Inlet 16 may have a filter suitable to prevent foreign material from entering air pump 22. Air pump 22 may be any type of air pump as known in the art. Air pump 22 directs a volume of air thorough an air channel 30 to heating center 24. A first valve 32 is present in air channel 30 and prevents air from flowing into heating center 24 from air pump 22 when not in use. Valve 32 may be adjustable, to regulate the flow of air, or act as a simple on/off valve.

Air channel 30 is in fluid communication with heating center 24. Heating center 24 has a chamber 20 for receiving vaporizable material 21 and a heater for vaporizing vaporizable material 21. Heating center 24 heats vaporizable material 21 to produce vapor 23 as is known in the art. Air supplied through air channel 30 mixes with vapor 23 to produce an inhalable vapor.

A second air channel 30 directs inhalable vapor from heating center 30 into vapor compressor 26. Vapor compressor 26 receives inhalable vapor, compresses it, and directs the compressed vapor to outlet 18. Vapor container 12 is secured to outlet 18 to receive the compressed vapor. Vapor container 12 may be attached, connected or secured to portable case 14 or vapor production system 10 during filling using common techniques such as a threaded connection or a twist lock connection. The compressed vapor contained within the vapor container 12 may then be used independently of the vapor production device 10. Additionally, the compressed vapor may be delivered at a much higher flow rate than what is available using a standard vaporizer. Because the vapor is compressed, vapor container 12 may hold a larger quantity than would otherwise be possible.

Control panel 28 is configured to control the components of the vapor control system. Control panel 28 regulates the power delivered to the heating center 24 to convert the vaporizable material to vapor at a temperature suitable for its vaporization. The suitable temperature may be entered manually by an operator, or control panel 28 may have stored data indicating an optimal temperature or heating profile. The quantity of air delivered to heating center 24 may be or is controlled by the control panel 28 and may be adjusted in combination with the power delivered to the heating center for optimal vapor production. Compressor 26 is further controlled by control panel 28 to deliver an optimal pressure for storage of the inhalable vapor. Depending on the type of vapor container and the vaporizable material being vaporized, compressor 26 regulates the level of compression to which the inhalable vapor is compressed.

Once vapor container 12 is filled with vapor mixture to a desired pressure, vapor container 12 may be detached or otherwise disconnected from portable case 14 or vapor production system 10.

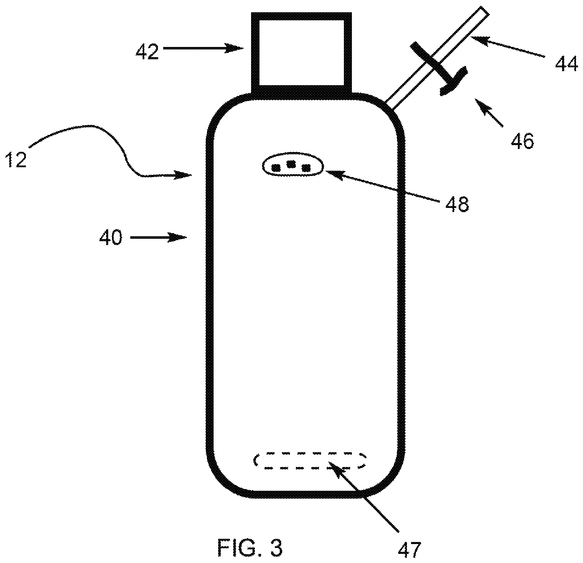

Vapor container 12 may be a small can shaped container having a neck for interfacing with the vapor production system 10, for example as illustrated in FIG. 3. Materials of construction or design of the receiving container are not critical so long as the container can safely withstand the pressure level of vapor output from system 10. Vapor container 12 is refillable and reusable. The container typically has a body 40 of sufficient durability and/or design to withstand pressures introduced during delivery of the compressed vapor. The container also has a connector portion 42 configured to be compatible with outlet 18. A vapor delivery device such as an inhalation tube or pipe may be connected to the neck of the vapor container 12. In other embodiments, vapor container 12 may have a separate connection or tube 44 for delivering pre-produced inhalation vapor. In certain other embodiments, the connection or tube for delivering pre-produced vapor may include a valve 46 or other regulating device to assist in delivery of vapor or to depressurize the container. In some embodiments, the interior of the vapor container 12 may be accessible for cleaning between fillings of different vaporizable materials. Alternatively, in other examples, the vapor container 12 may be sealed.

To prevent the vapor from condensing, or to reestablish the vapor properties within the container prior to inhalation, may require an establishing or maintenance of vapor temperature. To facilitate heat retention, the vapor container 12 may be insulated to inhibit the loss of heat from the vapor container 12, maintaining its temperature without external heating. Alternatively, vapor container 12 may be actively heated to prevent vapor from condensing. The vapor container 12 may have its own controller 48 including an integrated heater 47 for controlling the temperature of the vapor. Typically the heater is configured to maintain a pressurized vapor in a vapor state.

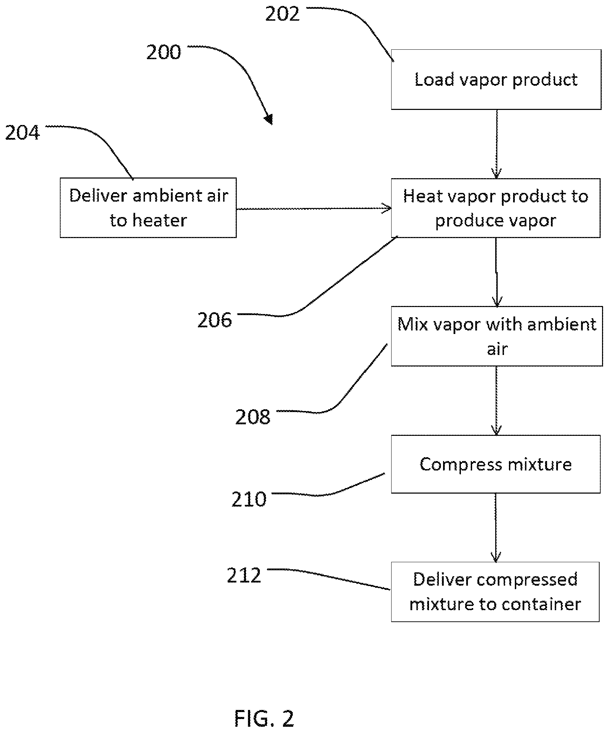

FIG. 2 illustrates a method 200 of producing inhalable vapor. In block 202, a vapor production device according to the invention, such as vapor production device 10 is loaded with a vapor vaporizable material, such as E-liquid. At block 204, ambient air is delivered to heating center 24. The ambient air is delivered to the heater by way of air pump 22. At block 206, the vaporizable material is heated to vaporize the material. Heating center 24 heats the vaporizable material to produce the vapor. The vapor is mixed with the supply of ambient air at block 208 and compressed at block 210. Compressor 26 is used to compress the mixture of ambient air and vapor. The compressed mixture of ambient air and vapor is then delivered to a suitable container at block 212, such as vapor container 12.

Some embodiments of the present invention are directed to vapor production device kits comprising a vapor production device according to the invention and instructions for operating the vapor production device. In some embodiments, the kit further comprises a container as described herein capable of storing pressurized vapor produced by the device.

## Claims

1. A vapor production device (10), comprising:
an air inlet (16);
an air pump (22) in fluid communication with the air inlet;
a heating center (24) in fluid communication with the air pump, the heating center having:
a chamber (20) configured to receive vaporizable material (21); and
a heater;
a compressor (26) in fluid communication with the heating center;
an outlet (18) in fluid communication with the compressor; and
a control panel (28),
wherein:
the air pump (22) is configured to supply ambient air to the heating center;
the heater is configured to heat the vaporizable material received in the chamber to a vaporization temperature to produce vaporized material;
the heating center is configured to supply the ambient air supplied by the air pump and the vaporized material produced by the heater to the compressor (26);
the compressor (26) is configured to compress the ambient air supplied by the heating center and the vaporized material supplied by the heating center to form a compressed mixture, and to deliver the formed mixture to the outlet;
the outlet (18) is configured to deliver the compressed mixture delivered by the compressor to a storage container; and
the control panel is configured to regulate power delivered to the heating center to convert vaporizable material to vaporized material at a vaporization temperature, and to control the compressor to deliver an optimal pressure for storage of the vaporized material, such that the compressor (26) regulates a level of compression at which the compressed mixture is formed depending on a type of the storage container and a type of the vaporizable material.

2. The vapor production device according to claim 1, further comprising a supply of vaporizable material.

3. The vapor production device according to any of claims 1-2, wherein the control panel (28) includes stored data indicating a heating profile.

4. The vapor production device according to any of claims 1-3, wherein the control panel is configured to control a quantity of the ambient air supplied to the heating center.

5. A system comprising:
the vapor production device according to any of claims 1-2; and
a storage container such that the outlet (18) of the vapor production device is configured to deliver the compressed mixture delivered by the compressor of the vapor production device to said storage container, wherein the storage container is suitable for storage of pressurized vapor produced by the vapor production device.

6. The system according to claim 5, wherein the storage container is insulated.

7. The system according to any of claims 5-6, wherein the storage container includes a heater configured to maintain a pressurized vapor stored in the storage container in a vapor state.

8. A method for producing inhalable vapor, comprising:
supplying a vaporizable material;
loading the vaporizable material into the chamber of the heating center of a vapor production device according to claim 1;
heating, with the heater of the heating center of the vapor production device, under the control of the control panel of the vapor production device, the loaded vaporizable material to a vaporization temperature to produce vaporized material;
delivering, with the air pump of the vapor production device, ambient air to the heating center, so that the delivered ambient air is mixed with vaporized material produced by the heater;
supplying the ambient air delivered to the heating center and the vaporized material produced by the heater to the compressor of the vapor production device;
compressing, with the compressor of the vapor production device, under the control of the control panel of the vapor production device, the supplied ambient air and the supplied vaporized material to form a compressed mixture; and
delivering the compressed mixture to the device outlet of the vapor production device.

9. The method for producing inhalable vapor according to claim 8, further comprising:
delivering, with the outlet of the vapor production device, the compressed mixture to a storage container for storage of said compressed vapor mixture.

10. A kit comprising:
a vapor production device according to any of claims 1 to 4; and
instructions for operating the vapor production device.

11. The kit according to claim 10, further comprising a storage container such that the outlet (18) of the vapor production device of the kit is configured to deliver the compressed mixture delivered by the compressor of the vapor production device of the kit to said storage container,
wherein the storage container is suitable for storage of pressurized vapor produced by the vapor production device.

12. The kit according to claim 11, wherein the storage container is insulated.

13. The kit according to any of claims 11-12, wherein the storage container includes a storage container heater configured to maintain a pressurized vapor contained in said storage container in a vapor state.

## Patentansprüche

1. Dampferzeugungsvorrichtung (10), umfassend:
einen Lufteinlass (16);
eine Luftpumpe (22) in Fluidverbindung mit dem Lufteinlass;
ein Heizzentrum (24) in Fluidverbindung mit der Luftpumpe, wobei das Heizzentrum aufweist:
eine Kammer (20), die dazu eingerichtet ist, verdampfbares Material (21) zu empfangen; und einen Heizer;
einen Kompressor (26) in Fluidverbindung mit dem Heizzentrum;
einen Auslass (18) in Fluidverbindung mit dem Kompressor; und
ein Bedienfeld (28),
wobei:
die Luftpumpe (22) dazu eingerichtet ist, dem Heizzentrum Umgebungsluft zuzuführen;
der Heizer dazu eingerichtet ist, das in der Kammer aufgenommene verdampfbare Material auf eine Verdampfungstemperatur zu erhitzen, um verdampftes Material zu erzeugen;
das Heizzentrum dazu eingerichtet ist, dem Kompressor (26) die von der Luftpumpe zugeführte Umgebungsluft und das von dem Heizer erzeugte verdampfte Material zuzuführen;
der Kompressor (26) dazu eingerichtet ist, die von dem Heizzentrum zugeführte Umgebungsluft und das von dem Heizzentrum zugeführte verdampfte Material zu komprimieren, um ein komprimiertes Gemisch zu bilden, und um das gebildete Gemisch an dem Auslass abzugeben;
der Auslass (18) dazu eingerichtet ist, das von dem Kompressor abgegebene komprimierte Gemisch an einen Aufbewahrungsbehälter abzugeben; und
das Bedienfeld dazu eingerichtet ist, Leistung, die an das Heizzentrum zur Umwandlung von verdampfbarem Material in verdampftes Material bei einer Verdampfungstemperatur abgegeben wird, zu regulieren und den Kompressor so zu steuern, dass ein optimaler Druck zur Lagerung des verdampften Materials bereitgestellt wird, so dass der Kompressor (26) ein Kompressionsniveau einstellt, bei dem das komprimierte Gemisch, in Abhängigkeit von einem Typ des Aufbewahrungsbehälters und einem Typ des verdampfbaren Materials, gebildet wird.

2. Dampferzeugungsvorrichtung gemäß Anspruch 1, die ferner eine Zuführung von verdampfbarem Material umfasst.

3. Dampferzeugungsvorrichtung gemäß einem beliebigen der Ansprüche 1-2, wobei das Bedienfeld (28) gespeicherte Daten enthält, die ein Heizprofil angeben.

4. Dampferzeugungsvorrichtung gemäß einem beliebigen der Ansprüche 1-3, wobei das Bedienfeld dazu eingerichtet ist, eine Menge der Umgebungsluft zu steuern, die dem Heizzentrum zugeführt wird.

5. System umfassend:
die Dampferzeugungsvorrichtung gemäß einem beliebigen der Ansprüche 1-2; und
einen Aufbewahrungsbehälter, wobei der Auslass (18) der Dampferzeugungsvorrichtung dazu eingerichtet ist, das von dem Kompressor der Dampferzeugungsvorrichtung abgegebene komprimierte Gemisch an diesen Aufbewahrungsbehälter abzugeben, wobei der Aufbewahrungsbehälter zur Lagerung von unter Druck stehendem Dampf, der von der Dampferzeugungsvorrichtung erzeugt wird, geeignet ist.

6. System gemäß Anspruch 5, wobei der Aufbewahrungsbehälter isoliert ist.

7. System gemäß der Ansprüche 5-6, wobei der Aufbewahrungsbehälter einen Heizer aufweist, der dazu eingerichtet ist, einen in dem Aufbewahrungsbehälter unter Druck gehaltenen Dampf in einem Dampfzustand zu halten.

8. Verfahren zum Erzeugen von inhalierbarem Dampf, umfassend:
Zuführen eines verdampfbaren Materials;
Laden des verdampfbaren Materials in die Kammer des Heizzentrums einer Dampferzeugungsvorrichtung gemäß Anspruch 1;
Heizen des geladenen verdampfbaren Materials mit dem Heizer des Heizzentrums der Dampferzeugungsvorrichtung unter der Kontrolle des Bedienfeldes der Dampferzeugungsvorrichtung auf eine Verdampfungstemperatur, um verdampftes Material zu erzeugen;
Zuführen von Umgebungsluft mit der Luftpumpe der Dampferzeugungsvorrichtung zu dem Heizzentrum, so dass die zugeführte Umgebungsluft mit verdampftem Material, das von dem Heizer erzeugt wird, vermischt wird;
Zuführen der an dem Heizzentrum zugeführte Umgebungsluft und des von dem Heizer erzeugten verdampften Materials zu demKompressor der Dampferzeugungsvorrichtung;
Komprimieren der zugeführten Umgebungsluft und des zugeführten verdampften Materials mit dem Kompressor der Dampferzeugungsvorrichtung unter der Kontrolle des Bedienfeldes der Dampferzeugungsvorrichtung, um ein komprimiertes Gemisch zu bilden; und
Abgeben des komprimierten Gemisches an den Vorrichtungsauslass der Dampferzeugungsvorrichtung.

9. Verfahren zum Erzeugen von inhalierbarem Dampf gemäß Anspruch 8, des Weiteren umfassend:
Abgeben des komprimierten Gemisches mit dem Auslass der Dampferzeugungsvorrichtung an einen Aufbewahrungsbehälter zum Speichern dieses komprimierten Dampfgemisches.

10. Kit, umfassend:
eine Dampferzeugungsvorrichtung gemäß einem beliebigen der Ansprüche 1 bis 4; und
Instruktionen zum Betreiben der Dampferzeugungsvorrichtung.

11. Kit gemäß Anspruch 10, ferner umfassend einen Aufbewahrungsbehälter, so dass der Auslass (18) der Dampferzeugungsvorrichtung des Kits dazu eingerichtet ist, das komprimierte Gemisch, das von dem Kompressor der Dampferzeugungsvorrichtung des Kits abgegeben wird, zu diesem Aufbewahrungsbehälter abzugeben,
wobei der Aufbewahrungsbehälter zur Speicherung von unter Druck stehendem Dampf, der von der Dampferzeugungsvorrichtung erzeugt wird, geeignet ist.

12. Kit gemäß Anspruch 11, wobei der Aufbewahrungsbehälter isoliert ist.

13. Kit gemäß einem der Ansprüche 11-12, wobei der Aufbewahrungsbehälter einen Aufbewahrungsbehälterheizer aufweist, der dazu eingerichtet ist, einen in diesem Aufbewahrungsbehälter enthaltenen, unter Druck stehenden Dampf in einem Dampfzustand zu halten.

## Revendications

1. Dispositif (10) de production de vapeur, comprenant :
une entrée d'air (16) ;
une pompe à air (22) en communication de fluide avec l'entrée d'air ;
un centre de chauffage (24) en communication de fluide avec la pompe à air, le centre de chauffage ayant : une chambre (20) configurée pour recevoir une matière vaporisable (21) ; et un dispositif de chauffage ;
un compresseur (26) en communication de fluide avec le centre de chauffage ;
une sortie (18) en communication de fluide avec le compresseur ; et
un panneau de commande (28),
dans lequel :
la pompe à air (22) est configurée pour fournir l'air ambiant au centre de chauffage ;
le dispositif de chauffage est configuré pour chauffer la matière vaporisable reçue dans la chambre jusqu'à une température de vaporisation pour produire une matière vaporisée ;
le centre de chauffage est configuré pour fournir l'air ambiant fourni par la pompe à air et la matière vaporisée produite par le dispositif de chauffage au compresseur (26) ;
le compresseur (26) est configuré pour comprimer l'air ambiant fourni par le centre de chauffage et la matière vaporisée fournie par le centre de chauffage pour former un mélange comprimé, et pour envoyer le mélange formé jusqu'à la sortie ;
la sortie (18) est configurée pour envoyer le mélange comprimé envoyé par le compresseur jusqu'à un réservoir de stockage ; et
le panneau de commande est configuré pour réguler une énergie envoyée jusqu'au centre de chauffage pour convertir la matière vaporisable en matière vaporisée à une température de vaporisation, et pour commander le compresseur pour fournir une pression optimale pour le stockage de la matière vaporisée, de telle sorte que le compresseur (26) régule un niveau de compression auquel le mélange comprimé est formé en fonction d'un type du réservoir de stockage et d'un type de la matière vaporisable.

2. Dispositif de production de vapeur selon la revendication 1, comprenant en outre une alimentation de matière vaporisable.

3. Dispositif de production de vapeur selon l'une quelconque des revendications 1 à 2, dans lequel le panneau de commande (28) inclut des données stockées indiquant un profil de chauffage.

4. Dispositif de production de vapeur selon l'une quelconque des revendications 1 à 3, dans lequel le panneau de commande est configuré pour commander une quantité de l'air ambiant fournie au centre de chauffage.

5. Système comprenant :
le dispositif de production de vapeur selon l'une quelconque des revendications 1 à 2 ; et
un réservoir de stockage tel que la sortie (18) du dispositif de production de vapeur est configurée pour envoyer le mélange comprimé envoyé par le compresseur du dispositif de production de vapeur jusqu'audit réservoir de stockage, dans lequel le réservoir de stockage est approprié au stockage de la vapeur sous pression produite par le dispositif de production de vapeur.

6. Système selon la revendication 5, dans lequel le réservoir de stockage est isolé.

7. Système selon l'une quelconque des revendications 5 à 6, dans lequel le réservoir de stockage inclut un dispositif de chauffage configuré pour maintenir une vapeur sous pression stockée dans le réservoir de stockage dans un état de vapeur.

8. Procédé pour produire une vapeur inhalable, comprenant :
la fourniture d'une matière vaporisable ;
le chargement de la matière vaporisable dans la chambre du centre de chauffage d'un dispositif de production de vapeur selon la revendication 1 ;
le chauffage, avec le dispositif de chauffage du centre de chauffage du dispositif de production de vapeur, sous le contrôle du panneau de commande du dispositif de production de vapeur, de la matière vaporisable chargée jusqu'à une température de vaporisation pour produire une matière vaporisée ;
l'envoi, avec la pompe à air du dispositif de production de vapeur, d'air ambiant jusqu'au centre de chauffage, de telle sorte que l'air ambiant envoyé est mélangé avec la matière vaporisée produite par le dispositif de chauffage ;
la fourniture de l'air ambiant envoyé au centre de chauffage et de la matière vaporisée produite par le dispositif de chauffage au compresseur du dispositif de production de vapeur ;
la compression, avec le compresseur du dispositif de production de vapeur, sous le contrôle du panneau de commande du dispositif de production de vapeur, de l'air ambiant fourni et de la matière vaporisée fournie pour former un mélange comprimé ; et
l'envoi du mélange comprimé à la sortie de dispositif du dispositif de production de vapeur.

9. Procédé pour produire une vapeur inhalable selon la revendication 8, comprenant en outre :
l'envoi, avec la sortie du dispositif de production de vapeur, du mélange comprimé jusqu'à un réservoir de stockage pour stockage dudit mélange de vapeur comprimé.

10. Trousse comprenant :
un dispositif de production de vapeur selon l'une quelconque des revendications 1 à 4 ; et
des instructions pour utiliser le dispositif de production de vapeur.

11. Trousse selon la revendication 10, comprenant en outre un réservoir de stockage tel que la sortie (18) du dispositif de production de vapeur de la trousse est configurée pour envoyer le mélange comprimé envoyé par le compresseur du dispositif de production de vapeur de la trousse jusqu'audit réservoir de stockage, dans lequel le réservoir de stockage est approprié au stockage de la vapeur sous pression produite par le dispositif de production de vapeur.

12. Trousse selon la revendication 11 dans lequel le réservoir de stockage est isolé.

13. Trousse selon l'une quelconque des revendications 11 à 12, dans lequel le réservoir de stockage inclut un dispositif de chauffage de réservoir de stockage configuré pour maintenir une vapeur sous pression stockée dans ledit réservoir de stockage dans un état de vapeur.
